# EUROPÄISCHE PATENTSCHRIFT

(11) **EP 1 330 981 B1**
(45) Veröffentlichungstag und Bekanntmachung des Hinweises auf die Patenterteilung: **02.11.2005**
(21) Anmeldenummer: 02027467.6
(22) Anmeldetag: 10.12.2002
(51) Int. Cl.: A61B 5/22, A61B 5/00

(54) **Anordnung zur Pulsmessung**
Heart rate monitor
Moniteur de la fréquence cardiaque

(30) Priorität: 26.01.2002 DE 20201125 U
(43) Veröffentlichungstag der Anmeldung: 30.07.2003
(73) Patentinhaber: Daum GmbH & Co. KG, 90587 Obermichelbach (DE)
(72) Erfinder: Daum, Wilhelm, 90768 Fürth (DE)
(74) Vertreter: Schneck, Herbert

(56) Entgegenhaltungen:
- EP-A- 0 590 634
- EP-A- 0 650 695
- EP-A- 1 101 511
- WO-A-97/14357
- US-A- 4 867 442

## Beschreibung

Die Erfindung richtet sich auf eine Anordnung zur Pulsmessung einer auf einem Standfahrrad bzw. Ergometer trainierenden Person gemäß den im Oberbegriff des Anspruchs 1 angegebenen Merkmalen.

Diese gattungsgemäße Art der Pulserfassung weist den grundsätzlichen Vorteil einer hohen Genauigkeit auf. Nachteilig ist demgegenüber, dass bei herkömmlichen Anordnungen eine Kabelverbindung zum Ohrläppchen hergestellt werden muß, was die Bewegungsfreiheit und das Wohlbefinden der trainierenden Person vermindert.

Es ist auch bereits bekannt, den Puls über einen Brustgurt abzugreifen, was es aber erforderlich macht, die Brust freizulegen, was umständlich ist und beim Trainieren in kühlerer Umgebung als unangenehm empfunden wird.

Letztlich ist es noch bekannt, die Pulsfrequenz über die Hände mittels Handelektroden abzugreifen, was zwar einerseits eine bequeme Messmethode ist, andererseits aber mit einer erheblichen Ungenauigkeit behaftet ist.

Aus der WO 97/14357 A1 ist eine Anordnung zur Pulsmessung einer Person bekannt. Die Pulsfrequenz wird dabei am Ohrläppchen abgegriffen. Die Anordnung umfasst eine entsprechende Messeinrichtung, die Pulsfrequenz-Signale an eine Anzeigeeinheit überträgt. Diese Anzeigeeinheit kann beispielsweise an einem Trainingsgerät angebracht sein. Nachteilig bei dieser bekannten Anordnung ist, dass sie kein optimales Training unterstützt bzw, ermöglicht.

Aus der EP 1 101 511 A2 ist ein Trainingsgerät zum Optimieren eines Trainings bekannt. Dieses umfasst eine Umwandlungseinrichtung, die erfasste Werte der körpereigenen Parameter in verbale Trainingsinformationen für den Trainierenden umwandelt und über eine Tonwiedergabeeinrichtung ausgibt.

Hiervon ausgehend liegt der Erfindung die Aufgabe zugrunde, eine Anordnung der gattungsgemäßen Art so zu verbessern, dass eine einfache und bequeme sowie beim Trainieren wenig störende Handhabung realisierbar ist. Außerdem soll ein leistungsgerechtes Training unterstützt werden.

Diese Aufgabe wird erfindungsgemäß durch die im kennzeichnenden Teil des Anspruchs 1 angegebenen Merkmale gelöst. Durch die erfindungsgemäße Ausgestaltung wird eine hohe Messgenauigkeit mit Komfort beim Trainieren kombiniert.

Insbesondere kann vorgesehen sein, dass die Sendeeinrichtung auf Infrarot-, Funk- oder Ultraschallbasis arbeitet.

Besonders bequem kann das Anlegen der Messeinrichtung dadurch gemacht werden, dass die Messeinrichtung an einer Art Kopfbedeckung, wie einem Kopfband, Mütze o. dgl. angeordnet ist, so dass sie beim Aufsetzen der Kopfbedeckung mehr oder weniger selbsttätig in den Bereich des Ohrläppchens gelangt und dort nur noch festgelegt zu werden braucht, z. B. in an sich bekannter Weise durch Anklipsen.

Zwischen der Empfangseinheit und der Sendeeinheit können Animationen, Informationen, Musik, Leistungsdaten o. dgl. übermittelt werden, die über einen an der Kopfbedeckung angebrachten Köpfhörer an die trainierende Person übertragbar sind.

Die Sendeeinheit kann beispielsweise mit einem Abspielgerät, wie einem Radio, einem CD-Spieler oder einem MP3-Spieler o.dgl. verbunden sein. Es entsteht somit keine Langeweile, während eines monotonen Konditionstrainings.

Es ist zweckmäßig, dass das Abspielen der Animationen abhängig von dem Pulsfrequenz-Signal erfolgt.

Das Training kann somit auf die Ambitionen bzw. die Leistungsfähigkeit der trainierenden Person eingestellt werden. Beispielsweise kann gewählt werden zwischen einem besonders leistungsfördernden oder gesunden Training.

Nachfolgend wird die Erfindung anhand eines bevorzugten Ausführungsbeispiels in Verbindung mit der Zeichnung näher erläutert. Dabei zeigen:
Fig. 1 eine schematische Darstellung einer auf einem Heimtrainingsgerät trainierenden Person, deren Pulsfrequenz mit der erfindungsgemäßen Pulsmessungs-Anordnung gemessen wird,

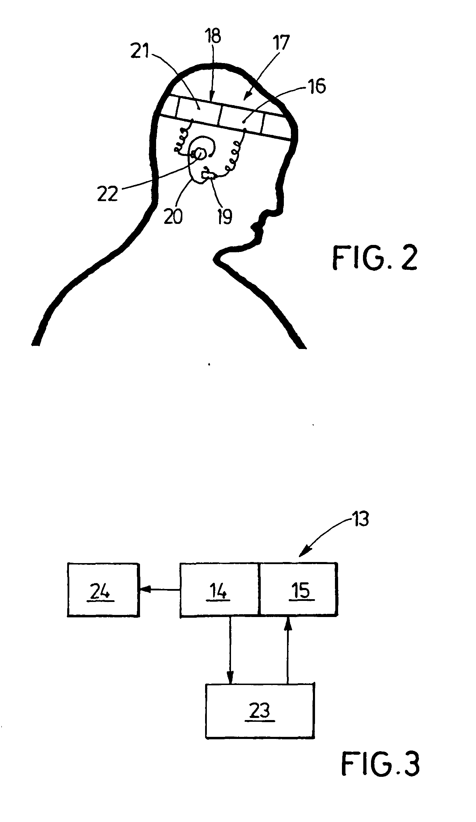

Fig. 2 eine vergrößerte Darstellung einer am Ohrläppchen angebrachten Messeinrichtung der Pulsmessungs-Anordnung, und
Fig. 3 ein Blockschaltbild der mit einer Konsole des Heimtrainingsgerät verbundenen Basiseinrichtung.

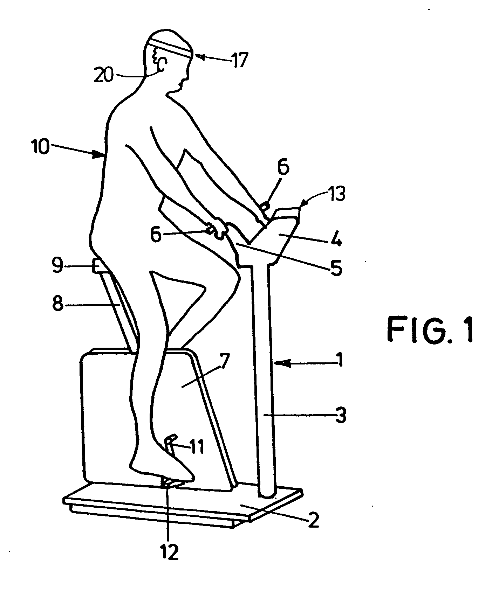

In Fig. 1 ist schematisch ein Heimtrainingsgerät in Form eines Standfahrrades 1 dargestellt, welches eine Grundplatte 2 umfasst, an welcher eine Standsäule 3 angeordnet ist. An der Oberseite der Standsäule 3 ist eine Anzeige-Konsole 4 vorgesehen. Seitlich der Anzeige-Konsole 4 erstreckt sich eine lenkerartige Anordnung mit zwei Handgriffen 6.

Hinter der Standsäule 3 ist ein Gehäuse 7 an der Grundplatte 2 angeordnet, von welchem sich eine Sitzsäule 8 mit einem Sitz 9 für eine trainierende Person 10 nach oben erstreckt. Weiterhin ist in dem Gehäuse 7 eine Tretkurbelanordnung 11 mit Fußpedalen 12 untergebracht.

Oberhalb der Anzeige-Konsole 4 ist im Sichtbereich der trainierenden Person 10 eine Basiseinrichtung 13 einer Pulsmessungs-Anordnung angebracht. Die Basiseinrichtung 13 kann auch in der Anzeige-Konsole 4 integriert sein und umfasst eine Empfängereinrichtung 14 sowie eine Sendeeinheit 15. Die Empfängereinrichtung 14 steht in Infrarotverbindung mit einer Sendeeinrichtung 16 einer Messeinrichtung 17, die in einem Kopfband 18 integriert ist. Mit der Sendeeinrichtung 16 ist ein Ohrclip 19 mit einem nicht dargestellten Infrarotlichtsensor verbunden.

Das Kopfband 18 wird während eines Trainings bzw. einer Pulsmessung von der trainierenden Person 10 getragen. Der Ohrclip 19 ist an einem Ohrläppchen 20 einer trainierenden Person 10 anzubringen. Der Infrarotlichtsensor durchleuchtet das Ohrläppchen 20 und ermittelt dadurch die Pulsfrequenz der trainierenden Person 10. Die Sendeeinrichtung 16 überträgt die ermittelte Pulsfrequenz an die Empfängereinrichtung 14 der Basiseinrichtung 13.

Die Messeinrichtung 17 weist außerdem eine Empfangseinheit 21 auf, die in Funkverbindung mit der Sendeeinheit 15 der Basiseinrichtung 13 steht. An einem Eingang der Sendeeinheit 15 ist ein als MP3-Spieler ausgebildetes Abspielgerät 23 angeschlossen, das Animationen, Informationen, Musik oder Leistungsdaten abspielt. Diese werden über einen Kopfhörer 22 an die trainierende Person 10 übertragen, der mit der Empfangseinheit 21 verbunden ist.

Das ermittelte Pulsfrequenz-Signal wird von der Empfänger-Einrichtung 14 einem Eingang des Abspielgeräts 23 zugeführt. Ein Ausgang der Empfängereinrichtung 14 ist außerdem mit einer Anzeigeeinrichtung 24 verbunden, die die ermittelte Pulsfrequenz anzeigt.

## Patentansprüche

1. Anordnung zur Pulsmessung einer auf einem Standfahrrad bzw. Ergometer (1) trainierenden Person (10), wobei die Pulsfrequenz am Ohrläppchen (20) abgegriffen wird, wobei der am Ohrläppchen (20) anzubringenden Messeinrichtung (17) eine Sendeeinrichtung (16) zugeordnet ist, die Signale entsprechend der Pulsfrequenz an eine Basiseinrichtung (13) mit einer Empfängereinrichtung (14) im Bereich einer Konsole (4) überträgt, **dadurch gekennzeichnet, dass**
- die Übertragung von Signalen zwischen der Messeinrichtung (17) und der Basiseinrichtung (13) bidirektional erfolgt,
- die Messeinrichtung (17) eine Empfangseinheit (21) aufweist, und
- die Basiseinrichtung (13) eine Sendeeinheit (15) aufweist.

2. Anordnung zur Pulsmessung nach Anspruch 1, **dadurch gekennzeichnet, dass** die Sendeeinrichtung (16) auf Infrarot-, Funk- oder Ultraschallbasis arbeitet.

3. Anordnung zur Pulsmessung nach Anspruch 1, **dadurch gekennzeichnet, dass** die Messeinrichtung (17) an einer Art Kopfbedeckung (18), wie einem Kopfband, Mütze o. dgl. angeordnet ist.

4. Anordnung zur Pulsmessung nach Anspruch 3, **dadurch gekennzeichnet, dass** an der Kopfbedeckung (18) ein Kopfhörer (22) zum Übertragen von Animationen, Informationen, Musik, Leistungsdaten o. dgl. angebracht ist.

5. Anordnung zur Pulsmessung nach Anspruch 4, **dadurch gekennzeichnet, dass** das Abspielen der Animationen abhängig von dem Pulsfrequenz-Signal erfolgt.

6. Anordnung zur Pulsmessung nach einem der vorherigen Ansprüche, **dadurch gekennzeichnet, dass** die Sendeeinheit (15) mit einem Abspielgerät (23) verbunden ist.

## Claims

1. Arrangement for measuring the pulse of a person (10) exercising on a stationary bicycle and ergometer (1), respectively, wherein the pulse frequency is piched up at the earlobe (20), wherein a transmitter (16) is arranged with the measuring device (17) to be attached to the earlobe (20), which transfers signals corresponding to the pulse frequency to a basic device (13) with a receiver (14), **characterized in that**
- the transmission of signals between the measuring device (17) and the basic device (13) is bi-directional,
- the measuring device (17) has a receiving unit (21), and
- the basic device (13) has a transmitting unit (15).

2. Arrangement for the pulse measurement according to claim 1, **characterized in that** the transmitter (16) works on a infrared, radio or ultrasonic basis.

3. Arrangement for the pulse measurement according to claim 1, **characterized in that** the measuring device (17) is disposed at a kind of headdress (18), like a headband, cap or the like.

4. Arrangement for the pulse measurement according to claim 3, **characterized in that** a headset (22) for transmitting animations, information, music, performance data or the like is disposed at the headdress (18).

5. Arrangement for the pulse measurement according to claim 4, **characterized in that** the play-back of the animations depends on the pulse frequency signal.

6. Arrangement for the pulse measurement according to one of the preceding claims, **characterized in that** the transmitting unit (15) is connected to a recorder (23).

## Revendications

1. Dispositif pour la mesure du pouls d'une personne (10) s'entraînant sur un vélo fixe ou un ergomètre (1), la fréquence d'impulsion étant prélevée sur le lobe de l'oreille (20), un dispositif d'émission (16) étant attribué au dispositif de mesure (17) à placer sur le lobe de l'oreille (20), lequel transmet des signaux en fonction de la fréquence cardiaque à un dispositif de base (13) équipé d'un dispositif récepteur (14) dans la zone d'une console (4), **caractérisé en ce que**
- la transmission de signaux s'effectue de façon bidirectionnelle entre le dispositif de mesure (17) et le dispositif de base (13),
- le dispositif de mesure (17) présente une unité de réception (21), et
- le dispositif de base (13) présente une unité d'émission (15).

2. Dispositif pour la mesure du pouls selon la revendication 1, **caractérisé en ce que** le dispositif d'émission (16) travaille à base d'infrarouge, de radio ou d'ultrason.

3. Dispositif pour la mesure du pouls selon la revendication 1, **caractérisé en ce que** le dispositif de mesure (17) est disposé sur une sorte de couvre-chef (18), comme un bandeau, une casquette ou similaires.

4. Dispositif pour la mesure du pouls selon la revendication 3, **caractérisé en ce qu'**un casque (22) pour la transmission d'animations, d'informations, de musique, de données de puissance ou similaires est placé sur le couvre-chef (18).

5. Dispositif pour la mesure du pouls selon la revendication 4, **caractérisé en ce que** le déroulement des animations s'effectue en fonction du signal de fréquence cardiaque.

6. Dispositif pour la mesure du pouls selon l'une quelconque des revendications précédentes, **caractérisé en ce que** l'unité d'émission (15) est reliée à un appareil de lecture (23).
